(19) Europäisches Patentamt
European Patent Office
Office européen des brevets

(11) **EP 0 543 999 B1**

(12) **EUROPEAN PATENT SPECIFICATION**

(45) Date of publication and mention
of the grant of the patent:
**07.10.1998 Bulletin 1998/41**

(21) Application number: **91914627.4**

(22) Date of filing: **15.08.1991**

(51) Int Cl.$^6$: **C09C 1/00**, C09C 1/40,
C09C 3/06, A61K 7/00

(86) International application number:
**PCT/JP91/01087**

(87) International publication number:
**WO 92/03119 (05.03.1992 Gazette 1992/06)**

(54) **FLAKY FINE POWDER, PRODUCTION THEREOF, AND COSMETIC**

FEINES, PLÄTTCHENFÖRMIGES PULVER, HERSTELLUNGSVERFAHREN, UND
KOSMETISCHES PRODUKT

POUDRE FINE ET LAMELLAIRE, SON PROCEDE DE PRODUCTION, ET PRODUIT COSMETIQUE

(84) Designated Contracting States:
**AT BE CH DE DK ES FR GB GR IT LI LU NL SE**

(30) Priority: **16.08.1990 JP 216403/90**

(43) Date of publication of application:
**02.06.1993 Bulletin 1993/22**

(73) Proprietor: **CATALYSTS & CHEMICALS
INDUSTRIES CO., LTD.
Kawasaki-shi, Kanagawa-ken 210-0913 (JP)**

(72) Inventors:
• **TANAKA, Hirokazu, 490-1-201, Futako
Takatsu-ku
Kanagawa 213 (JP)**

• **ENOMOTO, Naoyuki, 2-2, Kyounanmachi
Wakamatsu-ku
Fukuoka 808 (JP)**

(74) Representative: **Reitzner, Bruno, Dr.
Patentanwälte Dipl.-Ing. R. Splanemann
Dr. B. Reitzner, Dipl.-Ing. K. Baronetzky
Tal 13
80331 München (DE)**

(56) References cited:
**EP-A- 355 521          JP-A- 3 045 660
JP-A-58 149 959          JP-A-60 228 406
JP-A-62 072 514**

**Description**

[ Field of the Invention ]

The present invention relates to a flaky fine powder which is used as a compounding agent for cosmetic, pigment, and paint, or as a filler for such materials as plastics, and production thereof. Furthermore, the present invention relates to cosmetic comprising the aforesaid flaky fine powder.

[ Prior Art ]

A flaky fine powder has been used as a compounding agent for such materials as cosmetic, pigment, and paint, or as a filler for such materials as plastics, and as this type of flaky fine powder, that produced by covering a surface of a flaky substance such as mica as a base with metal oxide or other appropriate material is well known.

This type of flaky fine power is produced by precipitating hydroxide from aqueous solution of metallic salt under coexistence of a flaky substance as a base and depositing said hydroxide on the surface of the flaky substance.

However, a covering layer formed on the surface of the aforesaid flaky substance is a layer comprising primary particles and/or aggregated particles of the hydroxide and having uneven thickness. For this reason, it is extremely difficult to control a form or a thickness of covered particles.

In addition, a main purpose of production of the conventional type of flaky fine powder covered with oxides was to make use of physical characteristics specific to the oxides used for covering (such as a refractive index or a light transmittance), but a flaky fine powder for use of its function originated from the shape of covered particles had not been known.

By the way, in German Patent Laid Open Publication No. 3922178 which was laid open on January 17, 1991 after the priority date (August 16, 1990) of this PCT application, a minute flaky base which does not aggregate but has a high dispersibility, characterized in that said base contains spherical particles, each having a smaller diameter as compared to that of the minute flaky base, by at least 0.5 % by weight, is disclosed. And, it is described in this publication that, as the minute flaky base, that having a diameter in a range from at maximum 1 to 500 μm is suited, and that a diameter of the spherical particle should preferably be in a range from 0.05 to 50 μm.

It seems that the base can easily move because spherical particles act as a kind of ball bearings on the surface of the minute flaky substance.

Furthermore, in the aforesaid publication, a method of mixing a suspension of a minute flaky base with a suspension of spherical particles, each having a smaller diameter as compared to that of the base, then filtering, drying, and calcining, if necessary, the base is disclosed as a method of producing the aforesaid minute flaky base not aggregating and having a high dispersibility. Also it is described in the publication that, when preparing the mixture described above, it is important to fully mix the two suspensions by means of ultrasonic processing.

JP-A-60-228406 relates to a cosmetic comprising a flaky extender pigment coated with an inorganic silicon compound. It discloses a method of coating mica or the like with silica by dispersing the mica in an alcoholic solution of an organosilicon compound, such as ethyl silicate, adding an alkaline alcohol solution and hydrolyzing the organosilicon compound to produce silica hydrate.

Moreover, EP-A-355 521 relates to a mineral nucleus such as kaolin and mica coated with a substantially continuous substantially uniform coating of an an acitve paper pigment such as synthetic alkali metal silicates and precipitated silicas.

[ Disclosure of the Invention ]

Objects of the present invention include, by covering a surface of a flaky substance as a base with spherical particles, reducing the glossiness of the base caused by the irregular reflection of light on its surface and improving the slipperiness to provide new and at the same time useful flaky fine powder as well as production thereof.

The flaky fine powder according to the present invention comprises a flaky substance and spherical silica particles immobilized on its surface, or comprises the flaky substance and the silica particles furthermore immobilized by a hydrolysate of alkoxysilane and/or silica gel.

The flaky fine powder according to the present invention is produced by adding (a) an alkoxysilane and/or silicic acid solution to a dispersion containing a flaky substance and silica particles,

or (b) dispersing a flaky fine substance and spherical silica particles in a dispersion medium comprising an organic solvent and/or water to adhere said silica particles on the surface of said flaky substance, then adding an alkoxysilane and/or silicic acid solution to said dispersion;
to immobilize said silica particles on the surface of said flaky substance by hydrolyzing said alkoxysilane and/or gelling said silicic acid solution.

The production of the flaky powder according to the present invention is very simple and economical and the flaky fine powder is physically very stable and is available for various applications as described above.

[ Brief Description of the Drawings ]

Fig.1 is a drawing showing a preferred area of a dispersion when depositing silica particles on a flaky substance with the permittivity ( $\varepsilon$ ) [ - ] in the horizontal axis and the ion concentration (N) [ ppm ] in the vertical axis.

Fig.2 and Fig.3 are pictures showing the structure of particles on the surface of the flaky fine powders obtained in Example 1 and Example 21 respectively, while Fig.4 and Fig.5 are pictures showing the structure of particles on the surface of the flaky fine powders obtained in Example 1 and Example 21 respectively after the samples underwent the fixing test.

Fig.6 through Fig.10 are drawings of reflected light distributions showing scattering of reflected light when light is irradiated on samples A to E respectively.

[ Best Mode for Carrying out the Invention ]

Description is made in detail for the flaky fine powder according to the present invention and production thereof.

There is no restriction over a base in the present invention, as far as the substance is flaky or scalelike, and generally such materials as a natural mica group including common mica, biotite, and sericite, or synthetic mica, talc, platelike silica, platelike titania, and glass flakes are available.

Appropriate thickness of a flaky substance is allowable for each application, but generally a flaky substance having a thickness of approximately 1 $\mu$m or less is often used.

Silica particles deposited on a flaky substance are almost spherical, and an average particle size should preferably be approximately 5.0 $\mu$m or less, and particles having an average particle size in a range from 0.05 to 3 $\mu$ m are especially preferred. The silica particles may comprise either only $SiO_2$ or compound oxide or mixture of the main component with one or more components other than $SiO_2$ such as $Al_2O_3$, $TiO_2$, $ZrO_2$, MgO, ZnO, $CeO_2$, or $Fe_2O_3$.

There is no specific restriction over a method of preparing silica particles and a method of dispersing the silica particles in a dispersion medium, and the silica particles are available in any form of hydrosol, organosol, or powder.

An operation to deposit silica particles on a surface of a base is carried out in a dispersion.

As a dispersion medium, water or organic solvent such as alcohol, glycol, ester, ketone or aromatic hydrocarbon solvent can he used independently or in combination thereof.

The operation for depositing silica particles on a surface of a flaky substance can be carried out in any of the following three ways.

(1) A base is added in a dispersion with silica particles dispersed therein, and then the dispersion is stirred.
(2) Silica particles are added in a dispersion with a base dispersed therein, and then the dispersion is stirred.
(3) Silica particles and a base are added simultaneously or one by one in a dispersion, and then the dispersion is stirred.

It is preferable that a dispersion, in which a flaky substance and silica particles are mixed and the two substances coexist, has the permittivity ( $\varepsilon$ ) which is equal to or larger than 15 and equal to or smaller than 80 ( $15 \leqq \varepsilon \leqq 80$ ), and in addition that the ion concentration (N) of the sum of cations and anions in the dispersion is in the following range.

①When $\varepsilon$ is equal to 15, $200 \text{ ppm} \leqq N \leqq 5 \times 10^4$ ppm.
②When $\varepsilon$ is equal to 80, $3 \times 10^4 \text{ppm} \leqq N \leqq 2 \times 10^5$ ppm.
③When $\varepsilon$ is larger than 15 but smaller than 80, N within a quadrilateral area formed with A (15, 200), B (15, $5 \times 10^4$), C (80, $2 \times 10^5$) and D ( 80, $3 \times 10^4$) in the (X,Y) coordinate system with the permittivity ( $\varepsilon$ ) [ - ] in the X axis and the ion concentration (N) [ppm] in the Y axis

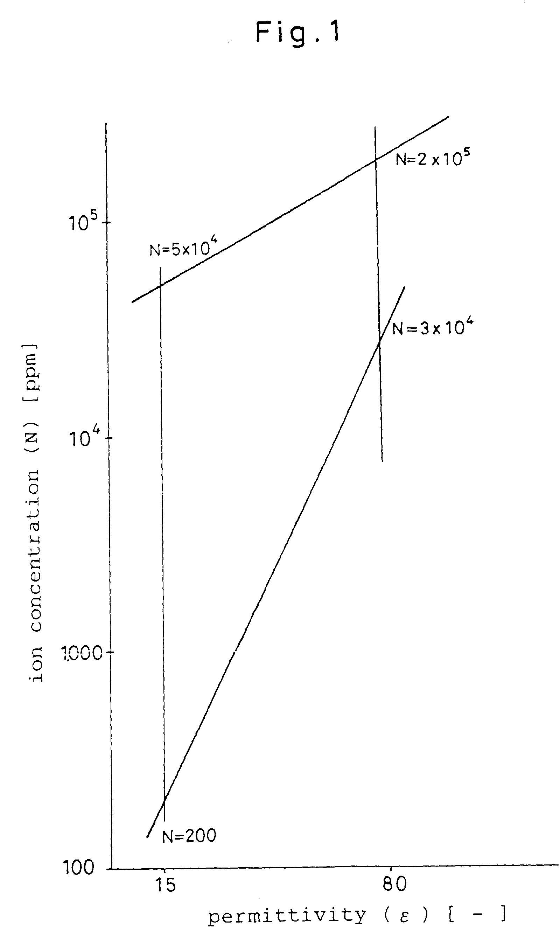

Fig.1 shows the area described above with the permittivity ( $\varepsilon$ ) [ - ] in the horizontal axis and the ion concentration (N) [ppm ] in the vertical axis.

If $\varepsilon$ and N are within this range, silica particles adhere on the flaky substance in the dispersion, and cover its surface almost with a single layer.

A permittivity of and an ion concentration (N) in a dispersion is controlled within this range by, for instance, deionizing the dispersion or adding ions of such substance as alkali.

It is not always required that particle size of silica particles is homogeneous in the processing for deposition. Even if particles with different diameter coexist, silica particles adhere without causing any specific problem, so long as the particle size is 5.0 µm or less. Also silica particles having a particle size of more than 5.0 µm adhere, but easily separate from a surface of a base. Also sometimes silica particles adhere on the surface of the base and form multiple layers there, but adhesive strength of silica particles other than those directly adhered on the surface of the base is extremely weak, so that the particles easily separate from the surface.

There is not specific restriction over a concentration of solids in a dispersion in which silica particles and a base are mixed, as far as the concentration does not cause any trouble to such operation as stirring, but preferably it should be not more than 70 % by weight.

In the present invention, it is not necessary that the entire surface of a base is covered with silica particles. A quantity of silica particles to be added against a quantity of a base is selected within a quantity of silica particles required to completely cover the entire surface of the base according to a purpose of each application.

Flaky fine powder covered with silica particles can be obtained by filtering, washing, and drying the dispersion after the aforesaid operation for deposition.

The flaky fine powder obtained as described above is in a state where silica particles have been adhered on the surface of the base, and not in a state where the silica particles have been immobilized to the surface. For this reason, if it is desired to flaky fine powder with silica particles tightly immobilized to the surface of the base, or when it is desired that silica particles hardly separate from the surface of the base so as, for instance, to achieve the soft focus effect in cosmetic, it is necessary to immobilize silica particles on the surface of the base.

In the present invention, a processing for immobilizing silica particles on a surface of a base is carried out by using alkoxysilane or silicic acid solution.

It should be noted that the processing for immobilizing silica particles may be carried out immediately after the aforesaid processing for deposition, and also that the processing for deposition and the processing for immobilizing silica particles may be carried out simultaneously.

When alkoxysilane is used as a fixing agent, alkoxysilane is added in the aforesaid dispersion, and then the alkoxysilane is hydrolyzed.

As alkoxysilane to be added, it is preferable to use tetraalkoxysilane. The tetraalkoxysilane is generally expressed by a chemical formula of $Si(OR)_4$, and the R indicates an alkyl group with a carbon number of 1 to 7. Concretely, tetramethoxysilane, tetraethoxysilane and tetraisopropoxysilane are included in this group.

A quantity of alkoxysilane to be added varies according to an adhesive strength of silica particles required in each application of flaky fine powder. Concretely, the quantity can be obtained by estimating a quantity of alkoxysilane in terms of $SiO_2$, required for a hydrolysate of alkoxysilane to fill clearance between silica particles deposited on a surface of a base with a certain thickness.

The adhesive strength of silica particles becomes stronger as a quantity of hydrolysate used to fill the clearance between silica particles increases, but even if the quantity exceeds a particle size of the silica particles, the adhesive strength does not become higher than a certain level.

There is no specific restriction over the condition of hydrolysis of tetraalkoxysilane, and it may be carried out under conventional conditions. For instance, a dispersion, in which tetraalkoxysilane has been added, is hydrolyzed at a temperature in a range from 20°C to 150 °C.

Then, to promote the hydrolysis of alkoxysilane, alkali or acid may be added as a catalyst. As an alkali catalyst, such a material as ammonia, hydroxide of alkali metal, or amines is available. Also as an acid catalyst, various types of organic acid or inorganic acid can be used.

As described above, an operation for immobilizing silica particles may be carried out by using silicic acid solution, and herein the silicic acid solution is an aqueous solution of a low grade polymer of silicic acid dealkalized by means of ion-exchanging an aqueous solution of alkali metal silicate such as water glass. If the dispersion medium is water itself or a percentage of water in it is high, the processing by using silicic acid solution is recommended.

The silicic acid solution is polymerized or gelled by adding a specified quantity of this silicic acid solution in a dispersion in which a flaky substance has been dispersed to fill clearance between silica particles with silica gel. Silica particles are immobilized to a surface of the base by the silica gel.

It should be noted that it is possible to carry out the processing for immobilizing silica particles by simultaneously using silicic acid solution and alkoxysilane.

Also it should be noted that the conditions concerning the permittivity ( $\varepsilon$ ) and the ion concentration (N) for a dispersion in the aforesaid processing for deposition are not applicable to the processing for immobilizing silica particles. Namely, so long as silica particles are immobilized to a surface of a base to some degree by means of operation for immobilizing them after adhesion, the dispersion may not always satisfy these conditions.

After the processing for immobilizing silica particles as described above is finished, flaky fine powder with silica particles deposited on a surface and simultaneously immobilized with hydrolysate of alkoxysilane and/or silica gel can be obtained by filtering, washing and drying the disperse phase. Furthermore, this flaky fine powder may be calcined, if necessary.

The flaky fine powder obtained thus can be compounded in cosmetic, in which powder is generally used, in the same way as that for compounding a conventional type of inorganic pigment in it, as it is, or after additional surface treatment with a known agent according to the necessity.

Examples of this type of cosmetic include cosmetic such as liquid foundation, powder foundation, cake foundation, stick foundation, face powder, lip stick, rouge, eye liner, eye shadow, eyebrow pencil, and in addition body powder, anhidrotics, anti-suntan cream, solid white powder, milk lotion, and lotion. These types of cosmetic are excellent in feeling when used for applying and spreading on skin as well as in cosmetic finishing effect such as so-called soft focus effect, for instance, to make wrinkles invisible.

The surface treatment described above is generally carried out by using silane coupling agent. Also, processing with such a material as silicone oil, fluorine-based oil containing perfluoro alkyl group, higher fatty acid or salt thereof, ester of higher fatty acid to improve the water resistance and sweat resistance, or processing with such a material as lecithin, hydrogenated lecithin, acylamino acid, or peptide is useful for this purpose.

A compounding ratio of the flaky fine powder against the cosmetic as described above is freely selected according to characteristics of each cosmetic, and the ratio is around 0 5 to 40 % by weight, for instance, in liquid foundation, around 1 to 80 % by weight in cake foundation, around 1.5 to 85 % by weight in powder foundation, around 1 to 95 % by weight in face powder, and around 0.5 to 20 % by weight in cream, and generally a range from around 0.5 to 95 % by weight, and preferably of 2 to 85 % by weight against the cosmetic as a whole is selected.

And, various types of components which are generally used in cosmetic, oils such as higher aliphatic alcohol, higher fatty acid, ester oil, paraffin oil, and wax; alcohol such as ethyl alcohol propylene glycol, sorbitol, and glucose; mucopolysaccharide, agents for maintaining humidity such as PCA salt or lactate, various types of surfactant including nonionic, cationic, anionic, and amphoteric ones; thickeners such as gum arabic, xanthan, polyvinyl pyrolidone, ethyl cellulose, carboxymethyl cellulose, carboxyvinyl polymer, metamoriphic or non-metamoriphic clay minerals; solvent such as ethyl acetate, acetone, toluene; inorganic or organic pigment or dyestuff; antioxidant such as BHT and tocopherol; water, chemicals, ultraviolet ray absorbent, pH buffer solution comprising salt of an organic acid or an inorganic acid; chelating agent, antiseptic agent, perfume or other materials are selected and mixed in the aforesaid cosmetic according to the necessity.

Detailed description was made above for a case where the flaky fine power is compounded in cosmetic, but the flaky fine powder according to the present invention is not limited to use in cosmetic, and can be used as a compounding agent for pigment or flat paint, or as a filler for such materials as plastic independently or in combination with other materials as described in the beginning of this specification.

Detailed description is made hereinafter with reference to related experiments, but it should be noted that these experiments are introduced only as examples. When producing the flaky fine powder, in addition to the conditions employed in the experiments described below, an average particle size of silica particles may be changed, and alkoxysilane other than tetraethoxylane or tetramethoxylane may be used. For this reason, the present invention should not limitedly be understood depending on results of these experiments. The scope of the present invention is defined by claims, and is not restricted by the text in this specification at all. Also, any deformation or change belonging to an equivalent claim in each claim shall be regarded as within the scope of the present invention.

Example 1

Mixed liquid was prepared by mixing 100 g of silica organosol prepared by dispersing $SiO_2$ particles, each having an average particle size of 0.6 μm, in monoethylene glycol as a dispersion medium with a $SiO_2$ concentration of 20 % by weight with 220 g of isopropanol. 80 g of natural mica having a thickness of 0.3 μm and aspect ratio of 100 was added in this mixed liquid, and then 500 g of ethanol was added, stirring the liquid at the room temperature, and additionally pH was adjusted to more than 9.5 by adding 28 % aqueous ammonia in the liquid to adhere silica particles on the surface of the mica.

The permittivity ( ε ), the ion concentration (N), and the concentration of solids of this dispersion are shown in Table 1 together with values in other examples described hereinafter.

In the examples of the present invention, the permittivity ( ε ) at 25 °C was obtained by measuring a refraction index (n) of the dispersion and calculating through the following equation, assuming that dielectric loss does not occur.

$$\varepsilon = n^2$$

In the experiments, a refraction index meter Model RX-1000 produced by ATAGO Co., Ltd. was used, and measurement was carried out at 25°C.

The ion concentration (N) in each dispersion was measured by centrifuging 50 ml of a dispersion for 30 minutes at 3000 rpm and sampling the supernatant.

Measurement of the concentration of ammonium ions was carried out by means of the Kjeldahl method, while measurement of the concentration of cations such as sodium ions, calcium ions, and aluminium ions was carried out by atomic absorption spectrometry (by using a polarized Zeeman atomic absorption photometer produced by Hitachi, Ltd., Model 180-80) and measurement of the concentration of anions such as chloride ions, sulfate ions, and nitrate ions was carried out by means of an ion chromatography (produced by DIONEX, 2010i).

Then, this dispersion was heated to 45°C, and tetraethoxysilane and 28 % aqueous ammonia were added simultaneously for 5 hours, maintaining the aforesaid temperature and pH. A quantity of added tetraethoxysilane was 13.3 g in terms of $SiO_2$, and a quantity of added aqueous ammonia was 153 g.

After addition of the materials was finished, the mixture was additionally stirred for 2 hours, the dispersion was filtered, washed and then dried at 100 °C, and furthermore calcined for 4 hours at 600 °C, and thus flaky fine powder with silica particles immobilized on the surface of the mica almost in a single layer was obtained.

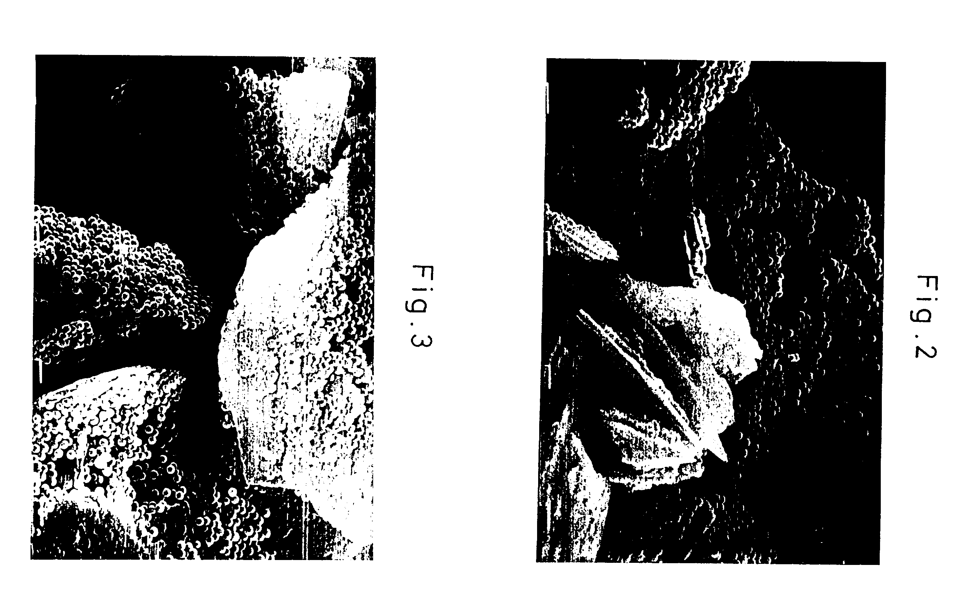

An electron microscopic picture of the flaky fine powder (magnified by 3500 times) is shown in Fig.2.

Example 2

The procedures were carried out in the same manner as described in Example 1 except that ethanol was used instead of isopropanol used in Example 1, and flaky fine powder was obtained. By electron microscope observation, it was confirmed that spherical silica particles were immobilized on the surface of the natural mica almost in a single layer similarly to Fig.2.

Example 3

The procedures were carried out in the same manner as described in Example 1 except that 1,3-butylene glycol was used instead of monoethylene glycol used in Example 1, and flaky fine powder was obtained. By electron microscope observation, it was confirmed that spherical silica particles were immobilized on the surface of the natural mica almost in a single layer similarly to Fig.2.

Example 4

The procedures were carried out in the same manner as described in Example 1 except that ethanol was used instead of monoethylene glycol used in Example 1, and flaky fine powder was obtained. By electron microscope observation, it was confirmed that spherical silica particles were immobilized on the surface of the natural mica almost in a single layer similarly to Fig.2.

Example 5

The procedures were carried out in the same manner as described in Example 1 except that tetramethoxysilane was used instead of tetraethoxysilane used in Example 1, and flaky fine powder was obtained. By electron microscope observation, it was confirmed that spherical silica particles were immobilized on the surface of the natural mica almost in a single layer similarly to Fig.2.

Example 6

The procedures were carried out in the same manner as described in Example 1 except that $SiO_2$ particles having an average particle size of 0.3 μm were used, and flaky fine powder was obtained. By electron microscope observation, it was confirmed that spherical silica particles were immobilized on the surface of the natural mica almost in a single layer similarly to Fig.2.

Example 7

The procedures were carried out in the same manner as described in Example 1 except that silica organosol in which $SiO_2$ particles having an average particle size of 1.0 μm were dispersed and which has a $SiO_2$ concentration of 60 % by weight was used, and flaky fine powder was obtained. By electron microscope observation, it was confirmed that spherical silica particles were immobilized on the surface of the natural mica almost in a single layer similarly to Fig.2.

Example 8

The procedures were carried out in the same manner as described in Example 1 except that 1,3-butylene glycol was used instead of monoethylene glycol used in Example 1, and that n-butanol was used instead of ethanol used in Example 1, and flaky fine powder was obtained. By electron microscope observation, it was confirmed that spherical silica particles were immobilized on the surface of the natural mica almost in a single layer similarly to Fig.2.

Example 9

The procedures were carried out in the same manner as described in Example 1 except that 1,3-butylene glycol was used instead of monoethylene glycol used in Example 1, and that acetone was used instead of ethanol used in Example 1, and flaky fine powder was obtained. By electron microscope observation, it was confirmed that spherical silica particles were immobilized on the surface of the natural mica almost in a single layer similarly to Fig.2.

Example 10

The procedures were carried out in the same manner as described in Example 1 except that silica organosol having a $SiO_2$ concentration of 40 % by weight was used, and that 60 g of natural mica was used without the isopropanol in Example 1, and flaky fine powder was obtained. By electron microscope observation, it was confirmed that spherical silica particles were immobilized on the surface of the natural mica almost in a single layer similarly to Fig.2.

Example 11

The procedures were carried out in the same manner as described in Example 1 except that the ethanol was not added in the mixed liquid, and that the liquid was heated to 35°C, and flaky fine powder was obtained. By electron microscope observation, it was confirmed that spherical silica particles were immobilized on the surface of the natural mica almost in a single layer similarly to Fig.2.

Example 12

The procedures were carried out in the same manner as described in Example 11 except that silica organosol having a $SiO_2$ concentration of 40 % by weight was used, and that 160 g of natural mica was used, and flaky fine powder was obtained. By electron microscope observation, it was confirmed that spherical silica particles were immobilized on the surface of the natural mica almost in a single layer similarly to Fig.2.

Example 13

Mixed liquid was prepared by mixing 200 g of silica hydrosol prepared by dispersing $SiO_2$ particles, each having an average particle size of 0.3 μm, in water as a dispersion medium with a $SiO_2$ concentration of 10 % by weight with 220 g of methanol. 80 g of talc having a thickness of 0.4 μm and aspect ratio of 120 was added in this mixed liquid, and then to adjust pH of more than 12.5, 28 % aqueous ammonia was added, stirring this liquid at the room temperature, to adhere silica particles on the surface of the talc.

Then, this dispersion was heated to 35°C, and maintaining the temperature and the aforesaid pH, the procedures for immobilizing by using tetraethoxysilane and 28 % aqueous ammonia were carried out in the same manner as described in Example 1, and thus flaky fine powder with silica particles immobilized on the surface of the talc almost in a single layer similarly to Fig.2 was obtained.

Example 14

Mixed liquid was prepared by mixing 100 g of silica organosol prepared by dispersing $SiO_2$ particles, each having an average particle size of 0.1 μm, in monoethylene glycol as a dispersion medium with a $SiO_2$ concentration of 20 % by weight with 220 g of isopropanol. 180 g of natural mica having a thickness of 0.3 μm and aspect ratio of 100 was added in this mixed liquid, and then 500 g of water was added, stirring this liquid at the room temperature, and additionally pH was adjusted to more than 11.0 by adding 28 % aqueous ammonia in the liquid to adhere silica particles on the surface of the mica.

Then, this dispersion was heated to 45°C, and maintaining the temperature and the aforesaid pH, the procedures for immobilizing by using tetraethoxysilane and 28 % aqueous ammonia were carried out in the same manner as described in Example 1, and thus flaky fine powder with silica particles immobilized on the surface of the mica almost in a single layer similarly to Fig.2 was obtained.

Example 15

The procedures were carried out in the same manner as described in Example 13 except that tetramethoxysilane was used instead of tetraethoxysilane used in Example 13, and flaky fine powder was obtained. By electron microscope observation, it was confirmed that spherical silica particles were immobilized on the surface of the talc almost in a single layer similarly to Fig.2.

Example 16

The procedures were carried out in the same manner as described in Example 11 except that 45 g ( as $SiO_2$ ) of tetraethoxysilane was used, and flaky fine powder was obtained. By electron microscope observation, it was confirmed that spherical silica particles were immobilized on the surface of the natural mica almost in a single layer similarly to Fig.2.

Example 17

Mixed liquid was prepared by mixing 100 g of silica organosol prepared by dispersing $SiO_2$ particles, each having an average particle size of 0.6 $\mu$m, in monoethylene glycol as a dispersion medium with a $SiO_2$ concentration of 20 % by weight with 220 g of isopropanol. 80 g of platelike silica having a thickness of 0.5 $\mu$m and aspect ratio of 90 was added in this mixed liquid, simultaneously with a mixed liquid comprising 13.3 g of tetraethoxysilane in terms of $SiO_2$ and 500 g of water, and additionally pH was adjusted to 11.0 by adding 28 % aqueous ammonia in this liquid, maintaining this pH and the temperature of 25 °C, the liquid was stirred for 2 hours.

After the aforesaid procedures, the dispersion was filtered, washed and then dried at 110 °C, and furthermore calcined for 4 hours at 600 °C, and thus flaky fine powder with silica particles immobilized on the surface of the platelike silica almost in a single layer similarly to Fig.2 was obtained.

Example 18

The procedures were carried out in the same manner as described in Example 17 except that tetraisopropoxysilane was used instead of tetraethoxysilane used in Example 17, and flaky fine powder was obtained. By electron microscope observation, it was confirmed that spherical silica particles were immobilized on the surface of the platelike silica almost in a single layer similarly to Fig.2.

Example 19

80 g of natural mica having a thickness of 0.3 $\mu$m and aspect ratio of 100 was added in a 100 g of silica hydrosol prepared by dispersing $SiO_2$ particles, each having an average particle size of 0.6 $\mu$m, in water as a dispersion medium with a $SiO_2$ concentration of 20 % by weight, and then 83 g of 17.1 % aqueous solution of sodium hydroxide was added, stirring the liquid at the room temperature, to adhere silica particles on the surface of the mica.

Then, 91 g of silicic acid solution with a $SiO_2$ concentration of 5 % by weight was added in the dispersion, and maintaining the temperature of 95 °C, the procedures were carried out in accordance with Example 1, and thus flaky fine powder with silica particles immobilized on the surface of the mica almost in a single layer similarly to Fig.2 was obtained.

Example 20

80 g of natural mica used in Example 19 and 67 g of silicic acid solution with a $SiO_2$ concentration of 5 % by weight were added simultaneously in 100 g of silica hydrosol used in Example 19, and then 246 g of 25 % aqueous solution of sodium hydroxide was added in the dispersion, the procedures were carried out in the same manner as described in Example 19, and thus flaky fine powder with silica particles immobilized on the surface of the mica almost in a single layer similarly to Fig.2 was obtained.

Example 21

Mixed liquid was prepared by mixing 100 g of silica organosol prepared by dispersing $SiO_2$ particles, each having an average particle size of 0.6 $\mu$m, in monoethylene glycol as a dispersion medium with a $SiO_2$ concentration of 20 %

by weight with 220 g of isopropanol. 80 g of natural mica used in Example 1 was added in this mixed liquid, and then 500 g of ethanol was added, stirring this liquid at the room temperature, and additionally pH was adjusted to more than 9. 5 by adding 28 % aqueous ammonia in the liquid to adhere silica particles on the surface of the mica.

After the aforesaid procedures, the dispersion was filtered and then dried at 110 °C , and thus flaky fine powder covered with silica particles was obtained.

An electron microscopic picture of the flaky fine powder (magnified by 3500 times) is shown in Fig.3.

Table 1

| Example No. | Permittivity [ - ] | Ion concn. [ ppm ] | Concn. of solids [ wt.% ] |
|---|---|---|---|
| 1 | 24.6 | 860 | 11.1 |
| 2 | 25.8 | 860 | 11.1 |
| 3 | 24.0 | 860 | 11.1 |
| 4 | 23.3 | 860 | 11.1 |
| 5 | 24.6 | 860 | 11.1 |
| 6 | 24.6 | 860 | 11.1 |
| 7 | 23.9 | 720 | 15.5 |
| 8 | 19.5 | 860 | 11.1 |
| 9 | 21.7 | 860 | 11.1 |
| 10 | 27.5 | 13730 | 14.5 |
| 11 | 31.4 | 50740 | 21.4 |
| 12 | 32.2 | 61780 | 35.7 |
| 13 | 55.1 | 65240 | 16.1 |
| 14 | 55.8 | 14380 | 19.1 |
| 15 | 55.1 | 65240 | 16.1 |
| 16 | 31.4 | 50640 | 21.4 |
| 17 | 25.2 | 13090 | 10.1 |
| 18 | 24.9 | 13070 | 10.0 |
| 19 | 78.5 | 49240 | 38.0 |
| 20 | 78.5 | 90120 | 21.0 |
| 21 | 24.6 | 860 | 11.1 |

( Fixing test of silica particles )

Water dispersion containing flaky fine powder obtained in Example 1 or Example 21 respectively, were stirred for about 10 hours by a mixer, and samples of the fine powder were observed by means of an electron microscope.

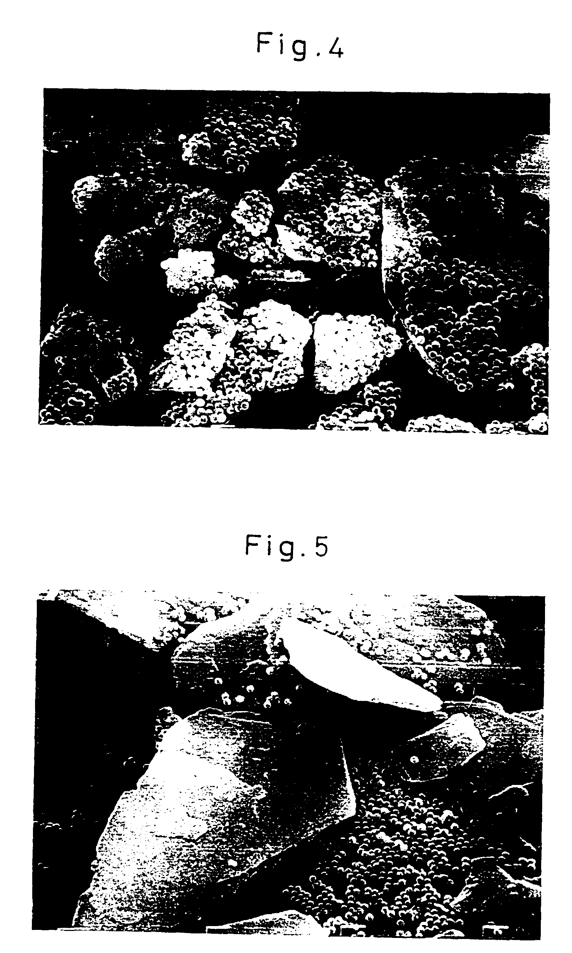

As to the flaky fine powder in Example 21, shown in Fig.5 ( magnified by 3500 times ), the greater part of silica particles was separated, while as to the flaky fine powder in Example 1, shown in Fig.4 ( magnified by 3500 times ), there were no change between the states before and after the stirring.

( Preparation of cosmetic )

Example 22

The flaky fine powder obtained in Example 1 and the following raw materials were compounded, and cake foundation was prepared.

| | |
|---|---|
| flaky fine powder | 35.0 wt.% |
| talc | 25.0 wt.% |
| sericite | 20.0 wt.% |
| titanium oxide pigment | 10.0 wt.% |
| iron oxide pigment (red) | 1.4 wt.% |
| iron oxide pigment (black) | 0.2 wt.% |
| iron oxide pigment (yellow) | 2.9 wt.% |
| stearyl alcohol | 2.0 wt.% |

(continued)

| lanoline | 2.0 wt.% |
|---|---|
| sorbitan fatty acid ester | 0.5 wt.% |
| triethanolamine | 1.0 wt.% |
| perfume | suitable |

First, stearyl alcohol, lanoline, sorbitan fatty acid ester, triethanolamine, and perfume were mixed and heated to 80 °C, and the rest of the powders mixed beforehand was added in the mixed liquid. This liquid was mixed sufficiently, then pressed and molded to cake foundation ( Sample A ).

Example 23

The procedures were carried out in the same manner as described in Example 22 except that the flaky fine powder obtained in Example 6 was used instead of that used in Example 22, and cake foundation was obtained ( Sample B ).

Example 24

The procedures were carried out in the same manner as described in Example 22 except that the flaky fine powder obtained in Example 7 was used instead of that used in Example 22, and cake foundation was obtained ( Sample C ).

Comparative Example 1

The procedures were carried out in the same manner as described in Example 22 except that the natural mica non-covered with silica particles ( used in Example 1 ) was used instead of the fine powder used in Example 22, and cake foundation was obtained ( Sample D ).

Example 25

The flaky fine powder obtained in Example 1 and the following raw materials were compounded, and milk foundation was prepared.

| flaky fine powder | 18.0 wt.% |
|---|---|
| pure water | 61.1 wt.% |
| triethanolamine | 1.1 wt.% |
| methyl para-hydroxybenzoate | suitable |
| carboxymethylcellulose | 0.2 wt.% |
| bentonite | 0.5 wt.% |
| stearic acid | 2.4 wt.% |
| propylen glycol monostearate | 2.0 wt.% |
| cetostearyl alcohol | 0.2 wt.% |
| liquid paraffin | 3.0 wt.% |
| liquid lanoline | 2.0 wt.% |
| isopropyl myristate | 8.5 wt.% |
| propyl para-hydroxybenzoate | suitable |
| titanium oxide pigment | 1.0 wt.% |
| perfume | suitable |

First, carboxymethylcellulose was dispersed into pure water, then bentonite was added, and the dispersion was stirred sufficiently and heated to 70 °C. Next, triethanolamine and methyl para-hydroxybenzoate were added in the dispersion, and a mixture comprising titanium oxide pigment and flaky fine powder was added, then the mixed liquid was stirred sufficiently by colloid mill and heated to 75 °C.

Another mixture comprising stearic acid, propylen glycol monostearate, cetostearyl alcohol, liquid paraffin, liquid lanoline, isopropyl myristate, and propyl para-hydroxybenzoate was prepared, and the mixture was heated to 80 °C, and stirred sufficiently with the above-mentioned mixed dispersion. Then, this mixture was cooled, adding perfume at 45°C, and stirred at the room temperature, and then milk foundation was obtained ( Sample E ).

(Evaluation of cosmetic)

Each of the samples A, B, C, D, and E described above was evaluated in terms of a degree of soft focus thereof as follows.

Each of the samples A to E was lightly applied to one face of a double-sided adhesive tape respectively, and the tape was adhered to a white-color plate to be used as a tested piece for measurement.

This test piece was set in a degital variable gloss meter (produced by Suga Shikenki, UGV-5D), light was irradiated onto a face on which the sample was applied at an incidence angle of 45 degrees, and scattering of reflected light was measured.

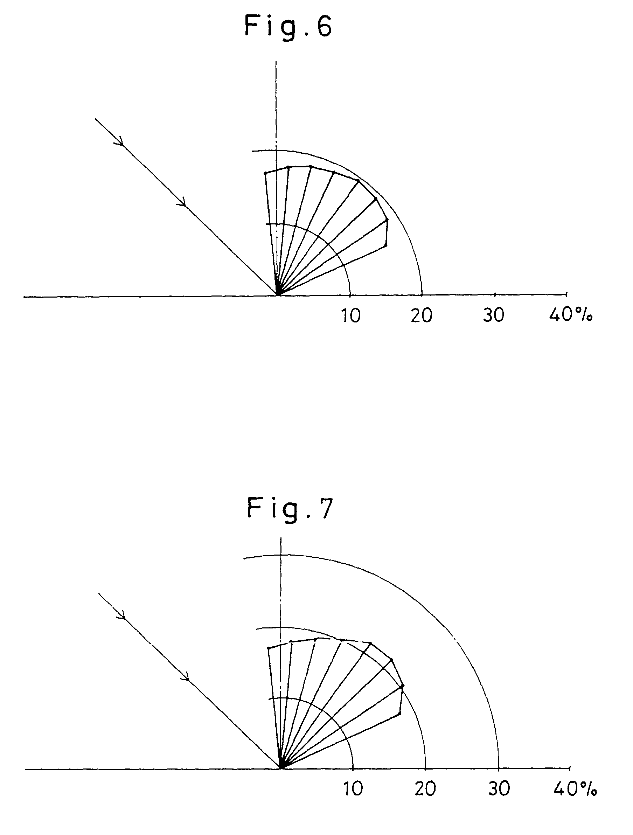

Fig.6 through Fig.10 were drawings showing distribution of reflected light for the sample A through E respectively, and a quantity of reflected light when light is injected at a certain angle to surface on a test piece is expressed with a length of a line segment as a relative value (%) against a quantity of reflected light from a reference plate (white-color plate on which no sample has been applied).

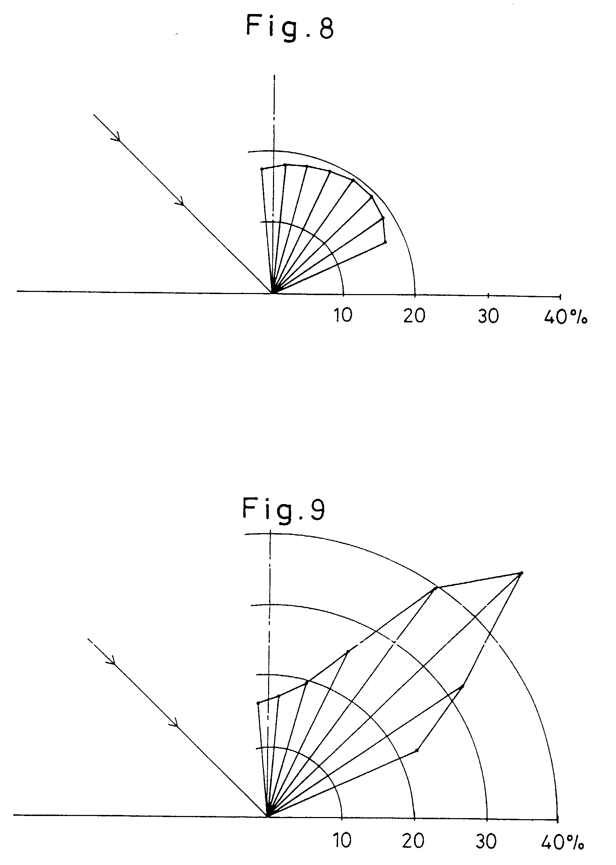

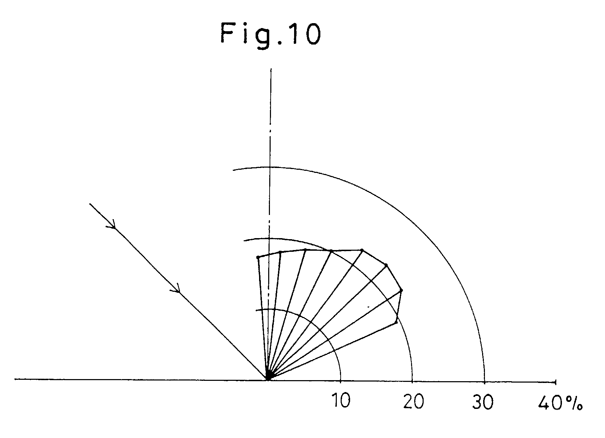

In any of sample A (Fig.6), sample B (Fig.7), sample C (Fig.8), and sample E (Fig.10), reflected light is distributed more homogeneously than in sample D (Fig.9), which indicates that the soft focus effect in these samples is more excellent than in sample D.

[ Industrial Applications ]

A surface of the flaky fine powder according to the present invention is not a layer comprising irregular particles as that of flaky fine powder according to the prior art, and is covered with spherical silica particles, so that the flaky fine powder has particular functions which are different from those of the conventional type of flaky fine powder. For this reason, the flaky fine power according to the present invention is useful as raw materials for such materials as a compounding agent for cosmetic and pigment, a filler for such materials as plastics, flat paint having low gloss, for providing a sense of high quality.

Especially, when used as a compounding agent for cosmetic, it can reduce excessive gloss of mica as a base and provide so-called the soft focus effect for, for instance, making wrinkles invisible. Also with this flaky fine powder according to the present invention, cosmetic having more excellent slipperiness and feelings as compared to the conventional type of cosmetic can be obtained.

**Claims**

1.  A method of producing a flaky fine powder, characterized in that :

    a) an alkoxysilane and/or silicic acid solution is added to a dispersion containing a flaky substance and silica particles, or

    b) a flaky fine substance and spherical silica particles are dispersed in a dispersion medium comprising an organic solvent and/or water to adhere said silica particles on the surface of said flaky substance, then an alkoxysilane and/or silicic acid solution is added to said dispersion;

    to immobilize said silica particles on the surface of said flaky substance by hydrolyzing said alkoxysilane and/or gelling said silicic acid solution.

2.  The method according to claim 1, characterized in that the permittivity ($\varepsilon$) of the dispersion is in the following range;

$$15 \leq \varepsilon \leq 80$$

    and in addition that the ion concentration (N) of the sum of cations and anions in said dispersion satisfies the following conditions,

    (1) 200 ppm $\leq$ N $\leq$ 5 X $10^4$ ppm, when $\varepsilon$ is 15,

    (2) 3 X $10^4$ ppm $\leq$ N $\leq$ 2 X $10^5$ ppm, when $\varepsilon$ is 80, and

    (3) N is in a quadrilateral area formed by A (15, 200), B (15, 5 X $10^4$), C (80, 2 X $10^5$) and D (80, 3 X $10^4$) in

the (X, Y) coordinate system with the X axis for the permittivity ($\varepsilon$) [-] and the Y axis for the ion concentration (N) [ppm], when $15 < \varepsilon$ 80.

3. A flaky fine powder comprising a flaky substance and spherical silica particles which are immobilized on the surface of the flaky substance.

4. The flaky fine powder according to claim 3, wherein said silica particles are immobilized on the surface of said flaky substance by a hydrolysate of alkoxysilane and/or silica gel.

5. A cosmetic comprising a flaky fine powder according to claims 3 or 4.


**Patentansprüche**

1. Verfahren zur Herstellung eines flockenförmigen feinen Pulvers, dadurch gekennzeichnet, daß

   a) eine Alkoxysilan- und/oder Kieselsäurelösung zu einer Dispersion gegeben wird, die eine flockenförmige Substanz und Kieselsäureteilchen enthält, oder

   b) eine flockenförmige feine Substanz und sphärische Kieselsäureteilchen in einem Dispersionsmedium dispergiert werden, welches ein organisches Lösungsmittel und/oder Wasser enthält, um die Kieselsäureteilchen auf den Oberflächen der flockenförmigen Substanz anzuheften, worauf der Dispersion eine Alkoxysilan- und/oder Kieselsäurelösung zugesetzt wird;

   um die Kieselsäureteilchen auf der Oberfläche der flockenförmigen Substanz durch Hydrolyse des Alkoxysilans und/oder durch Gelieren der Kieselsäurelösung auf der Oberfläche der flockenförmigen Substanz zu immobilisieren.

2. Verfahren nach Anspruch 1, dadurch gekennzeichnet, daß die Permittivität (Dielektrizitätskonstante) ($\varepsilon$) der Dispersion im folgenden Bereich liegt:

$$15 \leq \varepsilon \leq 80$$

und daß zusätzlich die Ionenkonzentration (N) der Summe der Kationen und Anionen in der Dispersion den folgenden Bedingungen genügt:

   (1) 200 ppm $\leq N \leq 5 \times 10^4$ ppm, wenn $\varepsilon = 15$,

   (2) $3 \times 10^4$ ppm $\leq N \leq 2 \times 10^5$ ppm, wenn $\varepsilon = 80$, und

   (3) N die vierseitige Fläche darstellt, die durch A (15, 200), B (15, $5 \times 10^4$), C (80, $2 \times 10^5$) und D (80, $3 \times 10^4$) im (X, Y)-Koordinatensystem gebildet wird, wobei die X-Achse für die Permittivität ($\varepsilon$) [-] und die Y-Achse für die Ionenkonzentration (N) [ppm] steht, wenn $15 < \varepsilon$ 80.

3. Flockenförmiges feines Pulver, enthaltend eine flockenförmige Substanz und sphärische Kieselsäureteilchen, die auf der Oberfläche der flockenförmigen Substanz immobilisiert sind.

4. Flockenförmiges feines Pulver nach Anspruch 3, worin die Kieselsäureteilchen auf der Oberfläche der flockenförmigen Substanz durch ein Hydrolysat von Alkoxysilan und/oder Kieselgel immobilisiert sind.

5. Kosmetikum, enthaltend ein flockenförmiges feines Pulver nach Anspruch 3 oder 4.


**Revendications**

1. Procédé de production d'une poudre fine en écailles, caractérisé en ce que :

a) on ajoute un alcoxysilane et/ou une solution d'acide silicique à une dispersion contenant une substance en écailles et des particules de silice, ou bien

b) on disperse une substance fine en écailles et des particules de silice sphériques dans un milieu de dispersion comprenant un solvant organique et/ou de l'eau, pour faire adhérer lesdites particules de silice à la surface de ladite substance en écailles, puis on ajoute à ladite dispersion un alcoxysilane et/ou une solution d'acide silicique,

pour immobiliser lesdites particules de silice sur la surface de ladite substance en écailles par hydrolyse dudit alcoxysilane et/ou gélification de ladite solution d'acide silicique.

2. Procédé selon la revendication 1, caractérisée en ce que la permitivité ($\varepsilon$) de la dispersion est comprise dans l'intervalle suivant :

$$15 \leq \varepsilon \leq 80$$

et, de plus, en ce que la concentration ionique (N) de la somme des cations et des anions se trouvant dans ladite dispersion satisfait aux conditions suivantes :

(1) 200 ppm $\leq$ N $\leq$ 5 x $10^4$ ppm, quand $\varepsilon = 15$,

(2) 3 x $10^4$ ppm $\leq$ N $\leq$ 2 x $10^5$ ppm, quand $\varepsilon = 80$, et

(3) N se trouve dans une zone quadrilatère formée des points A (15, 200), B (15, 5 x $10^4$), C (80, 2 x $10^5$) et D (80, 3 x $10^4$) dans le système de coordonnées (X, Y) dans lequel l'axe des X correspond à la permitivité ($\varepsilon$) [-] et l'axe des Y correspond à la concentration ionique (N) [ppm] quand 15 < $\varepsilon$ < 80.

3. Poudre fine en écailles comprenant une substance en écailles et des particules de silice sphériques qui sont immobilisées sur la surface de la substance en écailles.

4. Poudre fine en écailles selon la revendication 3, dans laquelle lesdites particules de silice sont immobilisées sur la surface de ladite substance en écailles par un hydrolysât d'alcoxysilane et/ou de gel de silice.

5. Produit cosmétique comprenant une poudre fine en écailles selon les revendications 3 ou 4.

# Fig.1

# Fig.2

# Fig.3

Fig. 4

Fig. 5

Fig.6

Fig.7

# Fig.8

10    20    30    40%

# Fig.9

10    20    30    40%

# Fig.10